# EUROPÄISCHE PATENTSCHRIFT

(11) **EP 0 645 368 B1**
(45) Veröffentlichungstag und Bekanntmachung des Hinweises auf die Patenterteilung: **05.02.1997**
(21) Anmeldenummer: 94114276.2
(22) Anmeldetag: 10.09.1994
(51) Int. Cl.: C07C 209/84

(54) **Verfahren zur Reinigung tertiärer Amine von primären und sekundären Aminen**
Process for the purification of tertiary amines from primary and secondary amines
Procédé pour purifier des amines tertiaires à partir d'amines primaires et secondaires

(30) Priorität: 20.09.1993 DE 4331840
(43) Veröffentlichungstag der Anmeldung: 29.03.1995
(73) Patentinhaber: BASF Aktiengesellschaft, 67063 Ludwigshafen (DE)
(72) Erfinder: Fuchs, Eberhard Dr., D-67227 Frankenthal (DE); Witzel, Tom Dr., D-67069 Ludwigshafen (DE); Stadler, Klaus Peter Dr., D-67434 Neustadt (DE)

(56) Entgegenhaltungen:
- EP-A- 0 413 259
- GB-A- 127 740

## Beschreibung

Die Erfindung betrifft ein Verfahren zur Verminderung des primären und sekundären Amingehalts in einem tertiären Amin durch Behandlung mit Carbonsäureestern.

Herstellungsbedingt finden sich in tertiären Aminen geringe Mengen an primären und sekundären Aminen. Destillativ ist eine Reinigung meist nur unter hohem Aufwand möglich, da die Siedepunktunterschiede häufig sehr gering sind. Ein Gehalt an sekundärem Amin ist aber durch die Gefahr der Nitrosaminbildung besonders unerwünscht.

In der DE-A-39 42 793 wird zur Vermeidung von primären oder sekundären Aminen ein Aldehydzusatz wie Formaldehyd oder Butyraldehyd empfohlen.

Diese Verfahren erlauben zwar den Anteil der unerwünschten Amine zu senken, das durch die Reaktion der NH-Gruppe mit dem Aldehyd gebildete Wasser läßt sich aber nur schwer destillativ entfernen. Außerdem findet bei Erhitzen des resultierenden Imins bzw. Enamins in Gegenwart des Wassers Rückspaltung zum primären oder sekundären Amin und dem Aldehyd statt.

Die DE-A-39 26 765 lehrt, den primären oder sekundären Amingehalt durch Zusatz eines organischen Anhydrids zu senken. Die Reaktion des Anhydrids mit der NH-Gruppe führt dann unter Carbonsäureabspaltung zu einem Amid. Nachteil dieses Verfahrens ist die Freisetzung der Carbonsäure, die mit dem tertiären Amin ein Salz bildet und somit zu Ausbeuteverlusten führt.

Nach der DD-A-298 503 werden zum Entfernen primärer oder sekundärer Amine Isocyanate angewandt. Dieses Verfahren weist jedoch den Nachteil auf, daß Isocyanate toxisch sind und somit nicht überstöchiometrisch zugesetzt werden sollten.

Der vorliegenden Erfindung lag daher die Aufgabe zugrunde, den zuvor genannten Nachteilen abzuhelfen.

Demgemäß wurde ein neues und verbessertes Verfahren zur Reinigung tertiärer Amine der allgemeinen Formel I 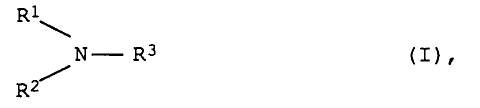 in der
- R¹,R², R³: C₁- bis C₂₀-Alkyl, C₂- bis C₂₀-Dialkylamino-C₂- bis C₁₀-alkyl, C₁- bis C₁₀-Alkoxy-C₂- bis C₁₀-alkyl, C₂- bis C₂₀-Hydroxyalkyl, C₃- bis C₁₂-Cycloalkyl, C₄- bis C₂₀-Cycloalkyl-alkyl, C₂- bis C₂₀-Alkenyl, C₄- bis C₃₀-Dialkylamino-alkenyl, C₃- bis C₃₀-Alkoxy-alkenyl, C₃- bis C₂₀-Hydroxyalkenyl, C₅- bis C₂₀-Cycloalkyl-alkenyl, gegebenenfalls durch C₁- bis C₈-Alkyl, C₂- bis C₈-Dialkylamino, C₁- bis C₈-Alkoxy, Hydroxy, C₃- bis C₈-Cycloalkyl, C₄- bis C₁₂-Cycloalkyl-alkyl ein bis fünffach substituiertes Aryl oder C₇- bis C₂₀-Aralkyl
bedeuten, von primären und sekundären Aminen, welches dadurch gekennzeichnet ist, daß man das zu reinigende Gemisch aus tertiärem, primärem und sekundärem Amin bei Temperaturen von 0 bis 200°C und Drücken von 1 bis 200 bar mit Carbonsäureestern der allgemeinen Formel II 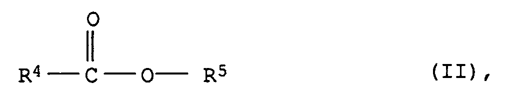 in der
- R⁴,R⁵: Wasserstoff, C₁- bis C₂₀-Alkyl, C₃- bis C₁₂-Cycloalkyl, C₄- bis C₂₀-Cycloalkyl-alkyl, gegebenenfalls durch C₁- bis C₈-Alkyl, C₃- bis C₈-Cycloalkyl, C₄- bis C₁₂-Cycloalkyl-alkyl ein bis fünffach substituiertes Aryl oder C₇- bis C₂₀-Aralkyl
bedeuten, behandelt.

Das erfindungsgemäße Verfahren läßt sich wie folgt durchführen:

Das zu reinigende Gemisch aus tertiärem Amin I, primärem und sekundärem Amin kann durch Zusatz von Carbonsäureestern II bei Temperaturen von 0 bis 200°C, bevorzugt 20 bis 150°C, besonders bevorzugt 30 bis 130°C und Drucken von 1 bis 200 bar, bevorzugt 1 bis 10 bar, besonders bevorzugt Atmosphärendruck (Normaldruck) behandelt werden.

Die sich dabei bildenden Alkylamide der primären und sekundären Amine können nach allgemeinen Reinigungsmethoden, beispielsweise destillativ, abgetrennt werden. Der bei der Reaktion außerdem freigesetzte Alkohol R⁵OH und überschüssiger Carbonsäureester (II) sind ebenfalls beispielsweise durch Destillation abtrennbar.

Nach Umsetzung der primären und sekundären Amine läßt sich das tertiäre Amin beispielsweise durch Destillation aus dem Reaktionsgemisch abtrennen.

In der Regel setzt man die Carbonsäureester II zu den primären und sekundären Aminen im Molverhältnis von 5 : 1 bis 1 : 1, bevorzugt 2 : 1 bis 1,1 : 1 ein.

Die Menge an primärem und sekundärem Amin kann beispielsweise gaschromatographisch oder durch Titration der aus primären und sekundären Aminen durch Umsatz mit Schwefelkohlenstoff erhaltenen Thiosäuren ermittelt werden.

Als Carbonsäureester II eignen sich insbesondere Carbonsäurealkylester wie C₁- bis C₂₀-Alkylcarbonsäure-C₁- bis C₂₀-alkylester, bevorzugt C₁- bis C₈-Alkylcarbonsäure-C₁- bis C₈-alkylester wie Buttersäuremethylester, Buttersäureethylester, Propionsäuremethylester, Propionsäureethylester, Essigsäuremethylester, Essigsäureethylester, Ameisensäuremethylester und Ameisensäureethylester, besonders bevorzugt Ameisensäuremethylester.

Die hohe Reaktivität des Ameisensäuremethylesters (Methylformiats) erlaubt die erfindungsgemäße Reinigung bereits bei niedrigen Temperaturen, ist aber auch bei höherer Temperatur durchzuführen.

Gemisch aus tertiärem Amin I, primärem und sekundärem Amin erhält man allgemein bei der Herstellung durch reduktive Alkylierung von primären Aminen mit Aldehyden oder Ketonen, bei der Nitrilhydrierung oder bei der Alkylierung von Aminen mit Alkoholen, z.B. bei der Umsetzung von Cyclohexylamin mit Methanol oder von Adipodinitril mit Dimethylamin.

Die Substituenten R¹, R², R³, R⁴ und R⁵ in den Verbindungen I und II haben folgende Bedeutungen:
- R¹,R²,R³,R⁴,R⁵: - C₁- bis C₂₀-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₁₂-Cycloalkyl, bevorzugt C₃- bis C₈-Cycloalkyl, besonders bevorzugt C₅- bis C₆-Cycloalkyl wie Cyclopentyl und Cyclohexyl,
- C₄- bis C₂₀-Cycloalkyl-alkyl, bevorzugt C₄- bis C₁₂-Cycloalkyl-alkyl, besonders bevorzugt C₄- bis C₈-Cycloalkyl-alkyl wie Cyclohexyl-methyl und Cyclohexyl-ethyl,
- Aryl wie Phenyl, 1-Naphthyl und 2-Naphthyl, bevorzugt Phenyl,
- C₇- bis C₂₀-Aralkyl, bevorzugt C₇- bis C₁₂-Phenylalkyl, besonders bevorzugt Benzyl, Phenylmethyl und Phenylethyl,
- durch C₁- bis C₈-Alkyl, C₂- bis C₈-Dialkylamino, C₁- bis C₈-Alkoxy, Hydroxy, C₃- bis C₈-Cycloalkyl, C₄- bis C₁₂-Cycloalkyl-alkyl ein bis fünffach substituiertes Aryl, durch C₁- bis C₄-Alkyl, C₂- bis C₄-Dialkylamino, C₁- bis C₄-Alkoxy, Hydroxy, C₅- bis C₈-Cycloalkyl, C₄- bis C₈-Cycloalkyl-alkyl ein bis dreifach substituiertes Phenyl wie Methylphenyl, Dimethylphenyl und Trimethylphenyl,
- durch C₁- bis C₈-Alkyl, C₂- bis C₈-Dialkylamino, C₁- bis C₈-Alkoxy, Hydroxy, C₃- bis C₈-Cycloalkyl, C₄- bis C₁₂-Cycloalkyl-alkyl ein bis fünffach substituiertes C₇- bis C₂₀-Aralkyl, durch C₁- bis C₄-Alkyl, C₂- bis C₄-Dialkylamino, C₁- bis C₄-Alkoxy, Hydroxy, C₅- bis C₈-Cycloalkyl, C₄- bis C₈-Cycloalkyl-alkyl ein- bis dreifach substituiertes C₇- bis C₂₀-Aralkyl wie 3,4-Dimethoxyphenylethyl,
- R¹,R²,R³: - C₂- bis C₂₀-Dialkylamino-C₂- bis C₁₀-alkyl, bevorzugt C₂- bis C₁₀-Dialkylamino-C₂- bis C₇-alkyl wie Dimethylaminoethyl, Dimethylaminopropyl, Dimethylaminobutyl, Dimethylaminopentyl, Dimethylaminohexyl, Dimethylaminoheptyl, Diethylaminoethyl, Diethylaminopropyl, Diethylaminobutyl, Diethylaminopentyl, Diethylaminohexyl, Diethylaminoheptyl, Dipropylaminoethyl, Dipropylaminopropyl, Dipropylaminobutyl, Dipropylaminopentyl, Dipropylaminohexyl, Dipropylaminoheptyl;
- C₁- bis C₁₀-Alkoxy-C₂- bis C₁₀-alkyl, bevorzugt C₁- bis C₆-Alkoxy, C₂- bis C₅-alkyl wie Methoxyethyl, Methoxypropyl, Ethoxyethyl, Ethoxypropyl, Propoxyethyl und Propoxypropyl,
- C₂- bis C₂₀-Hydroxyalkyl, bevorzugt C₁- bis C₈-Hydroxyalkyl, besonders bevorzugt C₁- bis C₄-Hydroxyalkyl wie Hydroxymethyl, 2-Hydroxyethyl, 2-Hydroxypropyl, 3-Hydroxypropyl,
- C₂- bis C₂₀-Alkenyl, bevorzugt C₂- bis C₈-Alkenyl, besonders bevorzugt C₂- bis C₄-Alkenyl wie Vinyl und Allyl,
- C₄- bis C₃₀-Dialkylamino-alkenyl, bevorzugt C₄- bis C₁₆-Dialkylamino-alkenyl, besonders bevorzugt C₄- bis C₁₂-Dialkylamino-alkenyl,
- C₃- bis C₃₀-Alkoxy-alkenyl, bevorzugt C₃- bis C₁₆-Alkoxy-alkenyl, besonders bevorzugt C₃- bis C₁₂-Alkoxy-alkenyl,
- C₃- bis C₂₀-Hydroxyalkenyl, bevorzugt C₂- bis C₈-Hydroxyalkenyl, besonders bevorzugt C₂- bis C₈-Hydroxyalkenyl,
- C₅- bis C₂₀-Cycloalkyl-alkenyl, bevorzugt C₅- bis C₁₂-Cycloalkyl-alkenyl, besonders bevorzugt C₅- bis C₁₂-Cycloalkyl-alkenyl,
- R⁴, R⁵: - Wasserstoff.

Die tertiären Amine I eignen sich als Lösungsmittel, Extraktionsmittel, Katalysatoren für FCKW-freie Polyurethanschäume, Katalysatoren für Epoxidharze, Korrosionsinhibitoren, Vorstufen für Bakterizide, Herbicide und Pharmazeutika (Kirk-Othmer, Encyclopedia of Chemical Technology, Vol. 2, 1978. 3rd Edition, J. Wiley + Sons, New York, S. 279 ff).

### Beispiele

### Beispiel 1

Zu einem Gemisch von 95 % Dimethylcyclohexylamin, 2,5 % Methylcyclohexylamin und 2,5 % Cyclohexylamin gibt man pro mol einer NH-Funktion 1,1 mol Methylformiat und erhitzt 2 h zum Sieden. Nach Destillation läßt sich das Dimethylcyclohexylamin mit einer Reinheit > 99,5 % in einer Destillationsausbeute > 97 % erhalten.

### Beispiel 2

Zu einem Rohgemisch von Tetramethylhexamethylendiamin, welches mit verschiedenen primären und sekundären Aminen verunreinigt ist, gibt man pro mol NH-Funktion 1,1 mol Methylformiat und erhitzt für 2 h auf 90°C. Nach Destillation läßt sich das Tetramethylhexamethylendiamin frei von primären und sekundären Aminen erhalten.

## Patentansprüche

1. Verfahren zur Reinigung tertiärer Amine der allgemeinen Formel I 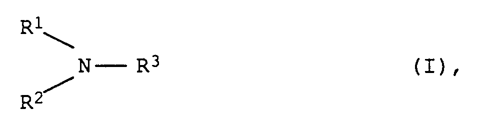 in der
R¹,R²,R³ C₁- bis C₂₀-Alkyl, C₂- bis C₂₀-Dialkylamino-C₂- bis C₁₀-alkyl, C₁- bis C₁₀-Alkoxy-C₂- bis C₁₀-alkyl, C₂- bis C₂₀-Hydroxyalkyl, C₃- bis C₁₂-Cycloalkyl, C₄- bis C₂₀-Cycloalkyl-alkyl, C₂- bis C₂₀-Alkenyl, C₄- bis C₃₀-Dialkylamino-alkenyl, C₃- bis C₃₀-Alkoxy-alkenyl, C₃- bis C₂₀-Hydroxyalkenyl, C₅- bis C₂₀-Cycloalkyl-alkenyl, gegebenenfalls durch C₁- bis C₈-Alkyl, C₂- bis C₈-Dialkylamino, C₁- bis C₈-Alkoxy, Hydroxy, C₃- bis C₈-Cycloalkyl, C₄- bis C₁₂-Cycloalkyl-alkyl ein bis fünffach substituiertes Aryl oder C₇- bis C₂₀-Aralkyl
bedeuten, von primären und sekundären Aminen, dadurch gekennzeichnet, daß man das zu reinigende Gemisch aus tertiärem, primärem und sekundärem Amin bei Temperaturen von 0 bis 200°C und Drucken von 1 bis 200 bar mit Carbonsäureestern der allgemeinen Formel II 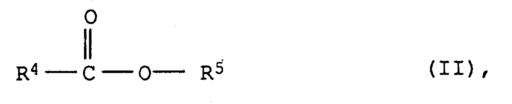 in der
R⁴,R⁵ Wasserstoff, C₁- bis C₂₀-Alkyl, C₃- bis C₁₂-Cycloalkyl, C₄- bis C₂₀-Cycloalkyl-alkyl, gegebenenfalls durch C₁-bis C₈-Alkyl, C₃- bis C₈-Cycloalkyl, C₄- bis C₁₂-Cycloalkyl-alkyl ein bis fünffach substituiertes Aryl oder C₇- bis C₂₀-Aralkyl
bedeuten, behandelt.

2. Verfahren zur Reinigung tertiärer Amine I nach Anspruch 1, dadurch gekennzeichnet, daß man Carbonsäureester II in der R⁴ Wasserstoff und R⁵ C₁- bis C₈-Alkyl bedeuten, einsetzt.

3. Verfahren zur Reinigung tertiärer Amine I nach Anspruch 1, dadurch gekennzeichnet, daß man als Carbonsäureester II Ameisensäuremethylester einsetzt.

4. Verfahren zur Reinigung tertiärer Amine I nach Anspruch 1, dadurch gekennzeichnet, daß man die Behandlung bei Temperaturen von 20 bis 150°C durchführt.

5. Verfahren zur Reinigung tertiärer Amine I nach Anspruch 1, dadurch gekennzeichnet, daß man die Behandlung bei Atmosphärendruck durchführt.

6. Verfahren zur Reinigung tertiärer Amine I nach Anspruch 1, dadurch gekennzeichnet, daß man die Carbonsäureester II zu den primären und sekundären Amin im Molverhältnis von 5 : 1 bis 1 : 1 einsetzt.

7. Verfahren zur Reinigung tertiärer Amine I nach Anspruch 1, dadurch gekennzeichnet, daß man die Carbonsäureester II zu den primären und sekundären Amin im Molverhältnis von 2 : 1 bis 1,1 : 1 einsetzt.

## Claims

1. A process for purifying tertiary amines of the general formula I 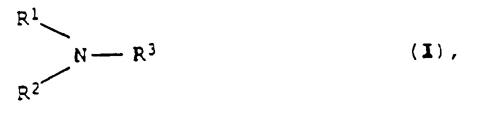 where
R¹, R² and R³ are C₁- to C₂₀-alkyl, C₂- to C₂₀-dialkylamino-C₂- to C₁₀-alkyl, C₁- to C₁₀-alkoxy-C₂- to C₁₀-alkyl, C₂- to C₂₀-hydroxyalkyl, C₃- to C₁₂-cycloalkyl, C₄- to C₂₀-cycloalkylalkyl, C₂- to C₂₀-alkenyl, C₄- to C₃₀-dialkylaminoalkenyl, C₃- to C₃₀-alkoxyalkenyl, C₃- to C₂₀-hydroxyalkenyl, C₅- to C₂₀-cycloalkylalkenyl, aryl which is unsubstituted or mono- to penta-substituted by C₁- to C₈-alkyl, C₂- to C₈-dialkylamino, C₁- to C₈-alkoxy, hydroxyl, C₃- to C₈-cycloalkyl or C₄- to C₁₂-cycloalkylalkyl, or C₇- to C₂₀-aralkyl,
from primary and secondary amines, which comprises treating the mixture of tertiary, primary and secondary amine to be purified at from 0 to 200°C and from 1 to 200 bar with carboxylic acid esters of the general formula II 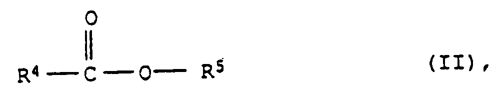 where
R⁴ and R⁵ are hydrogen, C₁- to C₂₀-alkyl, C₃- to C₁₂-cycloalkyl, C₄- to C₂₀-cycloalkylalkyl, aryl which is unsubstituted or mono- to pentasubstituted by C₁- to C₈-alkyl, C₃- to C₈-cycloalkyl, or C₄- to C₁₂-cycloalkylalkyl, or C₇- to C₂₀-aralkyl.

2. A process for purifying tertiary amines I as claimed in claim 1, wherein carboxylic acid esters II where R⁴ is hydrogen and R⁵ is C₁- to C₈-alkyl are employed.

3. A process for purifying tertiary amines I as claimed in claim 1, wherein the carboxylic acid ester II employed is methyl formate.

4. A process for purifying tertiary amines I as claimed in claim 1, wherein the treatment is carried out at from 20 to 150°C.

5. A process for purifying tertiary amines I as claimed in claim 1, wherein the treatment is carried out at atmospheric pressure.

6. A process for purifying tertiary amines I as claimed in claim 1, wherein the carboxylic acid esters II are employed in a molar ratio of from 5 : 1 to 1 : 1 to the primary and secondary amine.

7. A process for purifying tertiary amines I as claimed in claim 1, wherein the carboxylic acid esters II are employed in a molar ratio of from 2 : 1 to 1.1 : 1 to the primary and secondary amine.

## Revendications

1. Procédé de purification d'amines tertiaires de la formule générale I 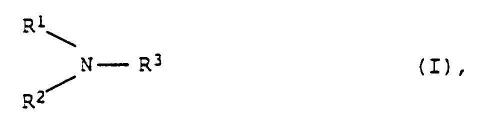 dans laquelle les symboles
R¹, R² et R³ représentent chacun un groupe alkyle en C₁ à C₂₀, dialkyle(C₂-C₂₀)aminoalkyle(C₂-C₁₀), alkoxy(C₁-C₁₀) alkyle(C₂-C₁₀)hydroxyalkyle en C₂ à C₂₀, cycloalkyle en C₃ à C₁₂, cycloalkyle(C₄-C₂₀)alkyle, alcényle en C₂ à C₂₀, dialkyl(C₄-C₃₀)aminoalcényle, alcoxy(C₃-C₃₀)alcényle, hydroxyalcényle en C₃ à C₂₀, cycloalkyle(C₅-C₂₀)alcényle, un radical aralkyle en C₇ à C₂₀ ou aryle, éventuellement de une à cinq fois substitué par des groupes alkyle en C₁ à C₈, dialkylamino en C₂ à C₈, alcoxy en C₁ à C₈, hydroxyle, cycloalkyle en C₃ à C₈, cycloalkyle(C₄-C₁₂) alkyle, d'amines primaires et secondaires, caractérisé en ce que l'on traite le mélange à épurer, constitué d'amines tertiaires, d'amines primaires et d'amines secondaires, à des températures de 0 à 200°C et sous des pressions de 1 à 200 bars par des esters d'acide carboxylique de la formule générale II 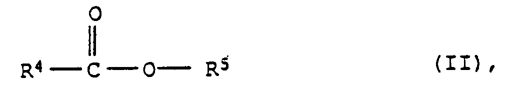 dans laquelle
les symboles R⁴ et R⁵ représentent chacun un atome d'hydrogène, un radical alkyle en C₁ à C₂₀, cycloalkyle en C₃ à C₁₂, cycloalkyle(C₄-C₂₀)alkyle, aralkyle en C₇ à C₂₀ ou aryle, éventuellement une à cinq fois substitué par des groupes alkyle en C₁ à C₈, cycloalkyle en C₃ à C₈, cycloalkyl(C₄-C₁₂)alkyle.

2. Procédé pour épurer des amines tertiaires suivant la revendication 1, caractérisé en ce que l'on utilise des esters d'acides carboxyliques II dans lesquels R⁴ représente un atome d'hydrogène et R⁵ représente un groupe alkyle en C₁ à C₈.

3. Procédé pour épurer des amines tertiaires I suivant la revendication 1, caractérisé en ce que l'on utilise, à titre d'ester d'acide caboxylique II, le formiate de méthyle.

4. Procédé pour épurer des amines tertiaires I suivant la revendication 1, caractérisé en ce que l'on entreprend le traitement à des températures de 20 à 150°C.

5. Procédé pour épurer des amines tertiaires I suivant la revendication 1, caractérisé en ce que l'on entreprend le traitement à la pression atmosphérique.

6. Procédé pour épurer des amines tertiaires I suivant la revendication 1, caractérisé en ce que l'on utilise les esters d'acides carboxyliques II par rapport à l'amine primaire et à l'amine secondaire dans la proportion molaire de 5:1 à 1:1.

7. Procédé pour épurer des amines tertiaires I suivant la revendication 1, caractérisé en ce que l'on utilise les esters d'acides carboxyliques II par rapport à l'amine primaire et à l'amine secondaire dans la proportion molaire de 2:1 à 1,1:1.
